(19) Europäisches Patentamt
European Patent Office
Office européen des brevets

(11) Veröffentlichungsnummer: **0 496 316 A1**

(12) # EUROPÄISCHE PATENTANMELDUNG

(21) Anmeldenummer: **92100818.1**

(51) Int. Cl.5: **A61K 9/00**

(22) Anmeldetag: **18.01.92**

(30) Priorität: **19.01.91 DE 4101540**

(43) Veröffentlichungstag der Anmeldung:
**29.07.92 Patentblatt 92/31**

(84) Benannte Vertragsstaaten:
**AT BE CH DE DK ES FR GB GR IT LI LU NL PT SE**

(71) Anmelder: **HOECHST AKTIENGESELLSCHAFT**
**Postfach 80 03 20**
**W-6230 Frankfurt am Main 80(DE)**

(72) Erfinder: **Röchling, Hans, Dr.**
**Geierfeld 25**
**W-6232 Bad Soden am Taunus(DE)**
Erfinder: **Düwel, Dieter, Dr.**
**Frankfurter Strasse 39**
**W-6238 Hofheim am Taunus(DE)**
Erfinder: **Schmid, Karlheinrich, Dr.**
**Zaunkönigweg 17 a**
**W-6238 Hofheim am Taunus(DE)**

(54) **Pour-on Formulierung zur Applikation von Anthelminthika.**

(57) Feinteilige Dispersionen anthelmithisch wirksamer Benzimidiazol- oder Benzthiazol-Derivate oder Pro-Benzimidazole in Wasser, in denen penetrationsfördernde Öle emulgiert sind, bzw. feinteilige Dispersionen der genannten Wirkstoffe in penetrationsfördernden Ölen als Pour-on Formulierung, Verfahren zu deren Herstellung sowie Verfahren zur Kontrolle von Helminthen bei Tieren.

Die neuen Formulierungen sind gut wirksam gegen im Verdauungstrakt und in Geweben parasitierende Nematoden und deren Stadien.

EP 0 496 316 A1

Die vorliegende Erfindung betrifft pour-on Formulierungen, Verfahren zu ihrer Herstellung und ihre Anwendung zur Kontrolle von Helminthen bei Tieren.

In einer Reihe von Publikationen wird das pour-on Verfahren auch "Spot on" oder "Aufgußverfahren" zur Bekämpfung von Ektoparasiten bei Tieren beschrieben: Journ. Econ. Entomology, 53, (1960), S. 814-817, EP-A- 045424, DE-A- 3208334, EP-A- 120286, EP-A- 311180. Hierbei wird die flüssige Zubereitung eines insektiziden/akariziden Wirkstoffes durch Aufgießen entlang der Rückenlinie vom Widerrist bis zur Schwanzwurzel bei landwirtschaftlichen Nutztieren, insbesondere Rindern, appliziert.

Die Formulierungen müssen so beschaffen sein, daß durch Spreizung eine Wirkung gegen die Schädlinge am gesamten Tierkörper erreicht wird.

Pour-on Formulierungen, die in der beschriebenen Applikationsweise auch gegen Endoparasiten wirksam sind, müssen systemisch aktiven Wirkstoff durch die Tierhaut in die Blutbahn transportieren. Solche Zubereitungen sind bekannt von Levamisol (EP-A- 137627, DE-A- 3212735, DE-A- 2614841) und von Ivermectin (EP 89302680). Beide Verbindungen sind in Wasser bzw. Lösungsmitteln gut löslich, so daß eine Penetration der Tierhaut relativ einfach erreicht werden kann.

Eine sehr wichtige Wirkstoffgruppe zur Bekämpfung von endoparasitischen Helminthen sind geeignet substituierte Benzimidazol- und Benzthiazol-Derivate sowie die Gruppe der sogenannten Pro-Benzimidazole (Verbindungen, die zu anthelminthisch wirksamen Benzimidazol-Derivaten metabolisiert werden können) wie die im folgenden aufgeführten Verbindungen der Formeln I bis III:

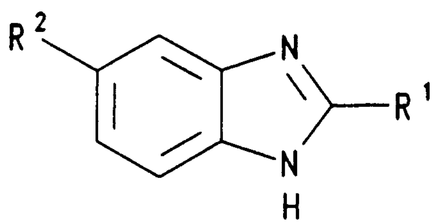

(I)

Ia) worin

- $R^1$ Methoxycarbonylamino bedeutet und
- $R^2$ n-Propylmercapto (Albendazol), Phenylmercapto (Fenbendazol), Phenylsulfinyl (Oxfendazol), Benzoyl (Mebendazol), p-Fluorbenzoyl (Flubendazol), p-Fluorphenylsulfonyloxy (Luxabendazol), Cyclopropylcarbonyl (Cyclobendazol), n-Butyl (Parbendazol), n-Propoxy (Oxibendazol) oder H (Carbendazim) bedeutet; oder

Ib) worin

- $R^1$ 4-Thiazolyl bedeutet und
- $R^2$ H (Thiabendazol) oder Isopropoxycarbonylamino (Cambendazol) bedeutet.

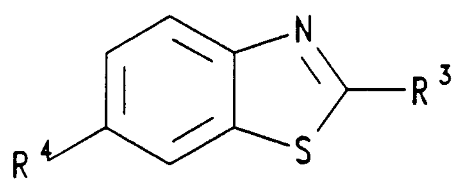

(II)

worin

- $R^3$ Methoxycarbonylamino bedeutet und
- $R^4$ n-Propoxy bedeutet (Tioxidazol).

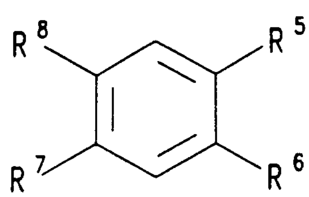

(III)

worin
$R^5$ $-N=C(NHCOOCH_3)_2$, $R^6$ $-NHCOCH_2OCH_3$, $R^7$ Phenylmercapto und
$R^8$ = H bedeuten (Febantel); oder
$R^5$

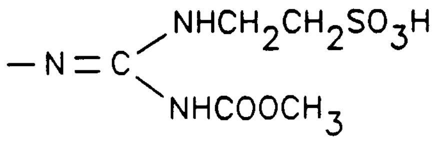

$R^6$ $NO_2$, $R^7$ = H und $R^8$ = n-Propylmercapto bedeuten
(Netobimin); oder
$R^5$ und $R^6$ jeweils $-NHCSNH-COOC_2H_5$, $R^7$ und $R^8$ jeweils H bedeuten (Thiophanat).

Bei diesen Wirkstoffen handelt es sich um schwer lösliche Substanzen, so daß es bisher nicht gelang, pour-on Formulierungen in geeigneten Lösungsmitteln herzustellen. Daher mußte bisher eine Behandlung Helminthen-infizierter Tiere (z.B. Rinder oder Schafe) im sogenannten Drench-Verfahren oder mit Hilfe eines Bolus oral durchgeführt werden.

Bei der Behandlung größerer Herden wäre die Anwendung des pour-on Verfahrens erheblich wirtschaftlicher, da es u .a. wesentlich weniger zeit- und arbeitsaufwendig ist. Die Tiere müssen nicht fixiert werden, die Gefährdung des Behandelnden durch Abwehrbewegung der Tiere ist geringer, außerdem ist der Streß, insbesondere für nicht handzahme Tiere, reduziert.

Es wurde überraschend gefunden, daß feinteilige Dispersionen (Suspensionen) der genannten anthelminthisch wirksamen Benzimidazole, Benzthiazole und Pro-Benzimidazole bei Verwendung geeigneter penetrationsfördernder Öle bei Applikation im pour-on Verfahren auf transdermalem Weg in den Organismus gelangen können, wo sie gute Wirkung gegen im Verdauungstrakt und in Geweben parasitierende Nematoden und deren Stadien entfalten. Hierzu ist bei geeigneter Dosierung lediglich das Aufbringen eines Streifens der Formulierung auf dem Rücken der Tiere erforderlich.

Die Erfindung betrifft daher anthelmintisch wirksame Zubereitungen, die als Wirkstoff mindestens ein anthelmintisch wirksames Benzimidazol, Benzthiazol oder Pro-Benzimidazol in feinteiliger Dispersion in einem penetrationsfördernden Mittel enthalten. Es können auch Gemische der genannten Wirkstoffe eingesetzt werden.

Die Wirkstoff-Teilchen in diesen Dispersionen sollen eine Korngröße von 0,05 bis ca. 30 $\mu$m, bevorzugt von 0,5 bis 10 $\mu$m aufweisen.

Als penetrationsfördernde Öle können Triglyceride gesättigter Pflanzenfettsäuren, z.B. Miglyol 812®, Triglycerindiisostearat oder Ester langkettiger Säuren, wie z.B. Oleyl-Oleat, Isopropylmyristat, Isopropylpalmitat, 2-Ethylhexylpalmitat, Isooctylstearat, Isopropylstearat, Laurinsäurehexylester oder Di-n-butyladipat eingesetzt werden; ferner langkettige Alkohole, wie z.B. Hexylalkohol, Octylalkohol, Decylalkohol, Oleylalkohol, 2-Octyldodecanol, 2-Hexyldecanol oder 2-Octyldecanol. Alle penetrationsfördernden Mittel können allein oder untereinander gemischt eingesetzt werden. Eine besonders gute penetrationsfördernde Wirkung haben langkettige Alkohole und unter diesen insbesondere die 2-alkylsubstituierten Alkohole. Die Öle werden in Gewichtsanteilen von 5 - 95 % eingesetzt, bevorzugt von 30 bis 90 Gew.-%. Die feinteiligen Wirkstoffe können auch in Wasser, in welchem penetrationsfördernde Öle emulgiert sind, dispergiert sein, wobei der Wassergehalt dieser Dispersionen bis zu 50 Gew.-%, vorzugsweise bis zu 30 Gew.-% betragen kann.

Als Emulgatoren und Netzmittel können verwandt werden: Alkylarylpolyglykoletheralkohole, z.B. ®Triton-Typen, ®Sapogenat-T-, ®Arkopal-Typen; Fettalkoholpolyglykolether wie z.B. ®Emulsogen M, A und MS12, ®Genapol X-080; Alkyl-diglykolethersulfat-Na-Salze wie z.B. ®Genapol LRO; Na-Laurylsulfat, z.B. ®Texapon K12; ethoxylierte Rizinusöl-Typen wie z.B. ®Emulsogen EL; $C_3$ bis $C_{20}$-Alkohol-Propylenoxid-block-oxalkylate wie z.B. Hoe S 3510 und HOE S 2436, Xylenoloxethylat mit 4-5 EO, Tristyrylphenol-Polyglykolether und Tristyrylphenol-Polyglykolether-phosphate wie z.B. Hoe S 3474 und Hoe S 3775, Alkylarylsulfonsäure Calcium- und Natrium-Salze wie z.B. Phenylsulfonat-Ca(70),Sorbitan-Fettsäureester, z.B. ®Span 60 und 80, Polyoxyethylen-Sorbitan-Fettsäureester, z.B. ®Tween 60 und 80 und Polyoxyethylenstearate, z.B. ®Myrj 45, 49 und 51.

Als Dispergatoren können u.a. eingesetzt werden: Ligninsulfonate wie z.B. ®Vanisperse CB und polymerisierte Arylalkylsulfonsäuren wie z.B. ®Darvan No. 3, Magnesium- und Aluminiumstearate, insbesondere Aluminium-monostearat. Die Hilfsmittel werden in Gewichtsanteilen von 0,5 bis 30 % eingesetzt, bevorzugt von 1 bis 10 %.

Als Entschäumungsmittel kann u.a. Silicon-Entschäumer SE2 verwandt werden.

Der Wirkstoffanteil in der Formulierung kann 1 bis 60 Gew.-% betragen, bevorzugt 5 - 20 Gew.-%.

Die Erfindung betrifft auch ein Verfahren zur Herstellung einer pour-on Formulierung, das dadurch gekennzeichnet ist, daß man die Wirkstoffe feinteilig in eine geeignete Darreichungsform bringt.

Zur Herstellung der pour-on Formulierungen können verschiedene Verfahren angewandt werden:

1. Der Wirkstoff aus der Klasse der Benzimidazole, Benzthiazole oder Pro-Benzimidazole wird unter Zusatz von Netzmitteln und Verwendung einer Perlmühle in Wasser fein vermahlen; das penetrationsfördernde Öl wird mit einem Emulgator vermischt und in der wäßrigen Wirkstoff-Dispersion emulgiert. Man erhält so eine Suspo-Emulsion, in der der Wirkstoff feinteilig in Wasser suspendiert ist und das penetrationsfördernde Öl darin emulgiert ist.

2. Der Wirkstoff wird unter Zusatz von Netz- und Dispergiermitteln und Verwendung einer Perlmühle in dem penetrationsfördernden Öl vermahlen.

3. Wenn der anthelminthisch wirkende Stoff in der beschriebenen Teilchenfeinheit vorliegt, sei es z.B. durch Verwendung geeigneter Fällungsmethoden bei der Herstellung oder durch Anwendung einer Trockenmahlung, so kann er auch nach Zugabe von Netz- und Dispergiermitteln ohne Naßmahlung im Wasser homogenisiert werden. Das Öl wird dann, wie unter 1. beschrieben, in der wäßrigen Dispersion emulgiert. Zur Trockenmahlung der Wirkstoffe kann eine Luftstrahlmühle, wie z.B. eine Spiralstrahlmühle verwendet werden.

4. Wenn der Wirkstoff, wie unter 3. beschrieben, in ausreichender Teilchenfeinheit vorliegt, so kann er auch ohne Naßmahlung bei Verwendung geeigneter Netz- und Dispergiermittel sowie geeigneter Misch- bzw. Rührapparaturen in dem penetrationsfördernden Öl dispergiert werden.

Die folgenden, mit Fenbendazol beispielhaft durchgeführten Herstellungsbeschreibungen sollen die Verfahren 1. bis 4. erläutern, ohne daß die Erfindung darauf beschränkt wäre.

Zur Naßmahlung der Wirkstoffe wurde eine Rührwerksmühle PE 075 von Netzsch-Feinmahltechnik GmbH verwandt.

Zur Homogenisierung mit schnellaufendem Rührer wurde ein Ultra Turrax®-Gerät von Janke und Kunkel eingesetzt.

Beispiel 1

7,5 %ige Fenbendazol pour-on Formulierung

In einer Rührwerksmühle wird Fenbendazol zusammen mit Netz- und Dispergiermitteln sowie mit einem Entschäumer vermahlen, bis 85 % der Teilchen kleiner als 1 µm sind, hierzu sind ca. 4 Stunden Mahlung erforderlich. Es werden Glasperlen mit einem Durchmesser von 2 mm verwandt, deren Mengenanteil doppelt so hoch ist, wie der des Wirkstoffes Fenbendazol:

1. Fenbendazol-Dispersion
40,00 Gew.-%, Fenbendazol, 99,8 %ig
4,00 Gew.-%, ®Genapol X-080
7,00 Gew.-%, ®Vanisperse CB
2,00 Gew.-%, ®Darvan Nr. 3
1,00 Gew.-%, Siliconentschäumer SE2
46,00 Gew.-% Wasser

Unter Rühren und leichtem Erwärmen auf 35 - 40 °C wird eine Lösung der Emulgatoren in den Ölen hergestellt:

2. Emulgatorlösung
9,38 Gew.-% Phenylsulfonat-Ca (70)
4,37 Gew.-% Hoe S 3510
6,25 Gew.-% Hoe S 3474-2
40,00 Gew.-% 2-Octyldodecanol
21,25 Gew.-% ®Miglyol 812

18,75 Gew.-% Fenbendazol-Dispersionen (1.) werden unter Rühren zu der Emulgator-Lösung gegeben. Es entsteht eine gut gießbare lagerstabile Suspo-Emulsion, die als pour-on Zubereitung verwendet werden kann.

Beispiel 2

10 %ige Fenbendazol pour-on Formulierung

Unter Verwendung von Glasperlen (Ø 2 mm) wird folgende Mischung in einer Rührwerksmühle vermahlen, bis 80 % der Teilchen kleiner als 1 µm sind:

1. Fenbendazol-Dispersion
35,00 Gew.-% Fenbendazol, techn. 99,8 %ig
4,00 Gew.-% ®Genapol X-080
7,00 Gew.-% ®Vanisperse CB
2,00 Gew.-% ®Darvan Nr. 3
1,00 Gew.-% ®Siliconentschäumer SE2
51,00 Gew.-% Wasser

Dann wird unter Rühren und Erwärmen auf 35 - 40 °C eine Lösung der Emulgatoren in den Ölen hergestellt:

2. Emulgatorlösung
9,38 Gew.-% Phenylsulfonat-Ca (70)
4,37 Gew.-% Hoe S 3510
6,25 Gew.-% Hoe S 3474-2
30,00 Gew.-% 2-Octyldodecanol
21,40 Gew.-% ®Miglyol 812

Zu dieser Emulgator-Lösung wird langsam unter Rühren 28,6 Gew.-% der wäßrigen Fenbendazol-Dispersion (1.) gegeben. Man erhält so eine gut gießbare, lagerstabile Fenbendazol pour-on Formulierung.

Beispiel 3

10 %ige Fenbendazol pour-on Formulierung

Wie in den Beispielen 1 und 2 beschrieben, wird eine Formulierung mit folgender Zusammensetzung hergestellt:

10,02 Gew.-% Fenbendazol, 99,8 %ig
1,00 Gew.-% ®Genapol X-080
1,75 Gew.-% ®Vanisperse CB
0,50 Gew.-% ®Darvan Nr. 3
0,25 Gew.-% Siliconentschäumer SE2
11,48 Gew.-% Wasser
9,37 Gew.-% Phenylsulfonat-Ca (70)
6,25 Gew.-% Hoe S 3474-2
4,38 Gew.-% Hoe S 3510
35,00 Gew.-% 2-Octyldodecanol
20,00 Gew.-% ®Miglyol 812

Die Formulierung ist gut gießbar und lagerstabil in bezug auf Homogenität und Wirkstoffstabilität.

Beispiel 4

Wie in den Beispielen 1 und 2 beschrieben, wird eine Fenbendazol-Dispersion 4 Stunden in einer Perlmühle gemahlen, bis 82 % der Teilchen kleiner als 1 µm sind:

1. Fenbendazol-Dispersion
40,00 Gew.-% Fenbendazol, techn. 99,8 %ig
4,00 Gew.-% ®Genapol LRO-Paste
56,00 Gew.-% Wasser

Dann wird unter Rühren und Erwärmen auf 35 - 40 °C eine Lösung der Emulgatoren in dem Gemisch von 2-Octyldodecanol und Miglyol 812® hergestellt:

2. Emulgatorlösung
9,38 Gew.-% Phenylsufonat-Ca (70)
4,37 Gew.-% Hoe S 3510
6,25 Gew.-% Hoe S 3474-2
35,00 Gew.-% 2-Octyldodecanol
20,00 Gew.-% ®Miglyol 812

25,00 Gew.-% der beschriebenen Fenbendazol-Dispersion (1.) werden langsam unter Rühren zu der Emulgator-Lösung gegeben.

Man erhält so eine gut applizierbare, Fenbendazol pour-on Formulierung.

Beispiel 5

Zunächst wird eine 40 %ige Fenbendazol-Dispersion in Wasser hergestellt. Mahldauer 4 Stunden, erreichte Teilchengröße: 80 % < 1 µm.

1. Fenbendazol-Dispersion
40,00 Gew.-% Fenbendazol, techn. 99,8 %ig
4,00 Gew.-% ®Genapol LRO-Paste
56,00 Gew.-% Wasser

25,0 Gew.-% der Fenbendazol-Dispersion (1.) wird unter Rühren zu 70,0 Gew.-% 2-Octyldodecanol gegeben; anschließend werden noch 5 % Wasser, ebenfalls unter Rühren, zugefügt.

Man erhält eine 10 %ige Fenbendazol pour-on Formulierung mit guter Gießfähigkeit und sehr guter Lagerstabilität.

Beispiel 6

Die Methode der Herstellung ist wie bei Beispiel 5 beschrieben, jedoch wird die Fenbendazol-Dispersion nur homogenisiert und nicht gemahlen, so daß der Wirkstoff Fenbendazol in der Korngröße zur Anwendung kommt, wie er bei der Herstellung anfällt: 50 % der Teilchen < 6 µm.

10 %ige Fenbendazol pour-on Formulierung
10,00 Gew.-% Fenbendazol, 99,98 %ig
1,00 Gew.-% ®Texapon K12
19,00 Gew.-% Wasser
70,00 Gew.-% 2-Octyldodecanol

Die Formulierung ist lagerstabil und gut applizierbar.

Beispiel 7

Methode der Herstellung wie bei Beispiel 5 beschrieben. Teilchenfeinheit Fenbendazol 80 % < 1 µm.

10 %ige Fenbendazol pour-on Formulierung
10,00 Gew.-% Fenbendazol, 99,98 %ig
1,00 Gew.-% ®Texapon K12
24,00 Gew.-% Wasser
25,00 Gew.-% ®Miglyol 812
40,00 Gew.-% 2-Octyldodecanol

Man erhält eine lagerstabile, gut applizierbare pour-on Formulierung.

Beispiel 8

Methode der Herstellung wie bei Beispiel 6 beschrieben. Teilchenfeinheit Fenbendazol 50 % < 6 µm.

10 %ige Fenbendazol pour-on Formulierung
10,00 Gew.-% Fenbendazol, 99,98 %ig
1,00 Gew.-% ®Texapon 12
24,00 Gew.-% Wasser
25,00 Gew.-% ®Miglyol 812
40,00 Gew.-% 2-Octyldodecanol

Die Formulierung hat eine gute Lagerstabilität und ist gut gießbar.

Beispiel 9

Methode der Herstellung wie bei Beispiel 4 beschrieben. Teilchenfeinheit 82 % < 1 µm.

10 %ige Fenbendazol pour-on Formulierung
10,00 Gew.-% Fenbendazol, 99,98 %ig
1,00 Gew.-% ®Genapol LRO
39,00 Gew.-% Wasser
15,00 Gew.-% Emulsogen A
35,00 Gew.-% 2-Octyldodecanol

Man erhält eine pour-on Formulierung von guter Lagerstabilität und guter Gießfähigkeit.

Beispiel 10

Methode der Herstellung wie bei Beispiel 4 beschrieben, jedoch erfolgt die Lösung des ®Span 60 in 2-Octyldodecanol bei 80 - 90° C. Teilchenfeinheit Fenbendazol 82 % < 1 µm.

10 %ige Fenbendazol pour-on Formulierung

10,00 Gew.-% Fenbendazol, 99,98 %ig
0,89 Gew.-% ®Texapon K12
21,33 Gew.-% Wasser
1,00 Gew.-% ®Span 60
66,78 Gew.-% 2-Octyldodecanol
Die Formulierung ist lagerstabil und gut applizierbar.

Beispiel 11

Zunächst wird ®Span 60 bei 80 - 90° C in 2-Octyldodecanol unter Rühren gelöst. Nach dem Abkühlen auf ca. 50° C wird Fenbendazol zugegeben; Korngröße: 50 % der Teilchen < 6 µm.

10 %ige Fenbendazol pour-on Formulierung

10,00 Gew.-% Fenbendazol, 99,98 %ig
10,00 Gew.-% ®Span 60
80,00 Gew.-% 2-Octyldodecanol
Die Mischung wird mit einem schnellaufenden Ultra Turrax®-Rührer homogenisiert.

Beispiel 12

Herstellung siehe Beispiel 11; Korngröße Fenbendazol: 50 % der Teilchen < 6 µm.

7,5 %ige Fenbendazol pour-on Formulierung

7,50 Gew.-% Fenbendazol, 99,98 %ig
10,00 Gew.-% ®Span 60
82,50 Gew.-% 2-Octyldodecanol
Die Mischung wird mit einem schnellaufenden Ultra Turrax®-Rührer homogenisiert.

Beispiel 13

Methode der Herstellung wie bei Beispiel 4 und 10 beschrieben. Korngröße Fenbendazol 50 % < 6 µm.

10 %ige Fenbendazol pour-on Formulierung

10,00 Gew.-% Fenbendazol, 99,98 %ig
1,00 Gew.-% ®Texapon K12
24,00 Gew.-% Wasser
1,00 Gew.-% ®Span 60
64,00 Gew.-% 2-Octyldodecanol
Es wird eine gut applizierbare Formulierung erhalten.

Beispiel 14

Aluminiummonostearat wird in 2-Octyldodecanol bei 140 - 150° C gelöst und 30 Minuten unter Rühren bei dieser Temperatur gehalten. Nach dem Abkühlen auf ca. 50° C wird Fenbendazol zugegeben und mit einem schnellaufenden Ultra-Turrax-Rührer homogenisiert.
Korngröße Fenbendazol: 50 % der Teilchen < 6 µm.

10 %ige Fenbendazol pour-on Formulierung

10,00 Gew.-% Fenbendazol, 99,98 %ig
1,00 Gew.-% Aluminiummonostearat
89,00 Gew.-% 2-Octyldodecanol
Es wird eine gut gießbare Suspension erhalten.

Beispiel 15

Aluminiummonostearat wird in 2-Octyldodecanol bei 140 - 150 °C gelöst und 30 Minuten unter Rühren bei dieser Temperatur gehalten. Nach dem Abkühlen auf ca. 100 °C wird ®Span 60 zugegeben und bei 50 °C Fenbendazol, beides ebenfalls unter Rühren. Anschließend wird mit einem schnellaufenden Ultra-Turrax-Rührer homogenisiert.
Korngröße Fenbendazol: 50 % der Teilchen < 6 µm.

10 %ige Fenbendazol pour-on Formulierung
10,00 Gew.-% Fenbendazol, 99,98 %ig
1,00 Gew.-% ®Span 60
2,50 Gew.-% Aluminiummonostearat
86,50 Gew.-% 2-Octyldodecanol
Es wird eine Formulierung mit guter Lagerstabilität und guter Gießfähigkeit erhalten.

Beispiel 16

Methode der Herstellung wie bei Beispiel 15 beschrieben
Korngröße Fenbendazol: 50 % der Teilchen < 6 µm.

20 %ige Fenbendazol pour-on Formulierung
20,00 Gew.-% Fenbendazol, 99,98 %ig
1,00 Gew.-% ®Span 60
1,50 Gew.-% Aluminiummonostearat
77,50 Gew.-% 2-Octyldodecanol

Beispiel 17

Methode der Herstellung wie bei Beispiel 15 beschrieben.
Korngröße Fenbendazol: 50 % der Teilchen < 6 µm.

11,37 %ige Fenbendazol pour-on Formulierung
11,37 Gew.-% Fenbendazol, 99,98 %ig ≙ 100 g/l
1,14 Gew.-% ®Span 60
2,85 Gew.-% Aluminiummonostearat
84,64 Gew.-% 2-Octyldodecanol

Beispiel 18

8,53 %ige Fenbendazol pour-on Formulierung
8,53 Gew.-% Fenbendazol, 99,98 %ig ≙ 75 g/l
1,14 Gew.-% ®Span 60
2,85 Gew.-% Aluminiummonostearat
87,48 Gew.-% 2-Octyldodecanol

Beispiel 19

5,75 %ige Fenbendazol pour-on Formulierung
5,75 Gew.-% Fenbendazol, 99,98 %ig ≙ 50 g/l
1,15 Gew.-% ®Span 60
2,88 Gew.-% Aluminiummonostearat
90,22 Gew.-% 2-Octyldodecanol
Die Präparate der Herstellungsbeispiele 16 bis 19 haben eine gute Lagerstabilität in bezug auf Phasentrennung und Konstanz des Wirkstoffgehaltes. Sie sind leicht gießbar und daher gut als pour-on Formulierung applizierbar.

Beispiel 20

11,37 %ige Fenbendazol pour-on Formulierung
11,37 Gew.-% Fenbendazol, 99,98 %ig ≙ 100 g/l
2,30 Gew.-% Span 60®
1,70 Gew.-% Aluminiummonostearat
84,62 Gew.-% 2-Octyldodecanol

Es wird verfahren, wie im Beispiel 15 beschrieben. Das verwendete Fenbendazol wird vorher in einer Spiralstrahlmühle so lange gemahlen, bis 50 % der Teilchen eine Korngröße von < 2,8 µm aufweisen.

Beispiele 21 - 26

Methode zur Herstellung siehe Beispiel 11
10,00 Gew.-% anthelminthischer Wirkstoff (siehe Tab. I)
10,00 Gew.-% ®Span 60
80,00 Gew.-% 2-Octyldodecanol
Bei Thiabendazol wurde eine 5 %ige Formulierung hergestellt:
5,00 Gew.-% Thiabendazol
10,00 Gew.-% ®Span 60
85,00 Gew.-% 2-Octyldodecanol

Tabelle I

| Beispiel | anthelminthischer Wirkstoff | allgemeine Formel |
|---|---|---|
| 21 | Oxibendazol | I a |
| 22 | Parbendazol | I a |
| 23 | Albendazol | I a |
| 24 | Mebendazol | I a |
| 25 | Thiabendazol | I b |
| 26 | Febantel | III |

Biologische Wirksamkeit

Die Untersuchungen erfolgten an Rindern, die artifiziell mit den wirtschaftlich bedeutsamen gastrointestinalen Nematoden der Wiederkäuer - Ostertagia ostertagi, Trichostrongylus axei, Trichostrongylus colubriformis und Cooperia oncophora - infiziert worden waren. Die Wirksamkeit wurde durch Sektion mit Bestimmung der Wurmbürde im Vergleich zu nicht behandelten Kontrolltieren ermittelt. Die Behandlung durch Aufgießen der Formulierungen entlang der Rückenlinie der Tiere erfolgt meist 14 Tage nach Infektion; zu diesem Zeitpunkt sind die Nematoden noch immatur und besonders schwer durch Anthelminthika zu beeinflussen.

Die Untersuchungen wurden in Übereinstimmung mit den Guidelines der WAAVP (World Association for the Advancement of Veterinary Parasitology; Powers, K.G. et al, Vet. Parasitol. (1982) 10, 265-284) durchgeführt.

In der folgenden Tabelle II sind einzelne Untersuchungen und Ergebnisse zusammengestellt.

Tabelle II

**Wirksamkeit von Fenbendazol in verschiedenen pour-on-Zubereitungen gegen immature Stadien gastrointestinaler Nematoden bei Rindern**

| Formulierung Beispiel Nr. | Dosis Fenbendazol mg/kg Körpermasse | Anzahl der Tiere | durchschnittliche prozentuale Reduktion der Wurmbürde im Vergleich zu den Kontrollen | | | |
|---|---|---|---|---|---|---|
| | | | Ostertagia ostertagi | Trichostrongylus | | Cooperia oncophora |
| | | | | axei | colubriformis | |
| 1 | 3,75 | 4 | 99 | 98 | 100 | 100 |
| | 7,50 | 4 | 72 | 94 | 100 | 94 |
| 2 | 3,75 | 4 | 98 | 99 | 98 | 100 |
| | 7,50 | 4 | 86 | 92 | 94 | 93 |
| 3 | 3,75 | 4 | 98 | 99 | 100 | 99 |
| | 7,50 | 4 | 99 | 100 | 100 | 100 |
| durchschnittl. Wurmbürde der Kontrollgruppe (n = 9) | | | 3922 | 480 | 132 | 274 |
| 4 | 7,50 | 3 | – | – | 100 | 99,8 |
| 5 | 7,50 | 3 | 78 | 88 | 100 | 100 |
| 6 | 7,50 | 3 | 91 | 80 | 100 | 100 |
| 7 | 7,50 | 3 | 36 | | 99 | 99 |
| 8 | 7,50 | 3 | 60 | 20 | 98 | 100 |
| 9 | 7,50 | 3 | 99 | 100 | 100 | 100 |
| durchschnittl. Wurmbürde der Kontrollgruppe (n = 3) | | | 2610 | 260 | 1067 | 1957 |
| 10 | 7,50 | 7 | 99 | 99 | 100 | 99 |
| 11 | 7,50 | 7 | 98 | 99 | 99 | 99 |
| durchschnittl. Wurmbürde der Kontrollgruppe (n = 7) | | | 6573 | 649 | 479 | 2279 |
| 11 | 5,0 | 7 | 82 | 92 | 100 | 99 |
| | 7,5 | 7 | 70 | 93 | 99 | 98 |
| 13 | 5,0 | 7 | 66 | 86 | 100 | 98 |
| | 7,5 | 7 | 91 | 99 | 99 | 99 |
| 14 | 5,0 | 7 | 68 | 97 | 99 | 99 |
| | 7,5 | 7 | 84 | 98 | 99 | 99 |
| 15 | 5,0 | 7 | > 80 | > 90 | > 98 | > 98 |
| | 7,5 | 7 | > 90 | > 95 | > 98 | > 98 |
| durchschnittl. Wurmbürde der Kontrollgruppe (n = 7) | | | 5763 | 490 | 1589 | 3867 |

In einem orientierenden Versuch wurde die Wirksamkeit von pour-on Formulierungen von Fenbendazol, Oxibendazol, Parbendazol, Albendazol, Mebendazol, Thiabendazol und Febantel (Herstellungsbeispiel 20 - 25) gegen Trichostrongylus colubriformis bei Nagern: Jirds (Meriones unguiculatus) festgestellt.

Die Dosierung betrug jeweils 0,2 ml pro Tier bzw. 0,4 ml bei Thiabendazol und bei Fenbendazol zusätzlich 0,1 und 0.05 ml; dieses Volumen wurde auf den geschorenen Nackenbereich der Jirds aufgetropft. Durch die Lokalisation der Applikationsstelle und die Einzelhaltung der Tiere wurde die Möglichkeit, Wirkstoff durch Belecken aufzunehmen, unterbunden.

Die Ergebnisse sind in Tabelle III aufgelistet.

Tabelle III

| Wirksamkeit von 'pour-on' Formulierungen gegen T. colubriformis in Jirds | | | | | | | |
|---|---|---|---|---|---|---|---|
| Wirkstoff | Herstell Beispiel | Konzentration | appl.Menge (ml/Tier) | Dosis mg/kg | Anzahl Würmer (x) | Anzahl Tiere | Wirkung in % |
| Fenbendazol | 11 | 10% | 0,2 | ca. 300 | 0 | 11 | 100 |
| Fenbendazol | 11 | 10 % | 0,1 | ca. 150 | 0 | 5 | 100 |
| Fenbendazol | 11 | 10 % | 0.05 | ca. 75 | 0 | 4 | 100 |
| Albendazol | 23 | 10 % | 0,2 | ca. 300 | 0 | 5 | 100 |
| Febantel | 26 | 10 % | 0.2 | ca. 300 | 0 | 5 | 100 |
| Mebendazol | 24 | 10 % | 0,2 | ca. 300 | 0 | 5 | 100 |
| Oxibendazol | 21 | 10 % | 0,2 | ca. 300 | 0 | 5 | 100 |
| Parbendazol | 22 | 10 % | 0,2 | ca. 300 | 0 | 5 | 100 |
| Thiabendazol | 25 | 5 % | 0,4 | ca. 300 | 0 | 5 | 100 |
| Kontrolle ohne Behandlung | | - | - | - | 67 | 9 | - |

**Patentansprüche**

1. Anthelminthisch wirksame Zubereitung, enthaltend als Wirkstoff mindestens ein anthelminthisch wirksames Benzimidazol, Benzthiazol oder Pro-Benzimidazol in feinteiliger Dispersion in einem penetrationsfördernden Mittel.

2. Mittel gemäß Anspruch 1, worin die Wirkstoffteilchen in der Dispersion eine Korngröße von 0,05 - 30 µm aufweisen.

3. Mittel gemäß Anspruch 1 oder 2, worin der Wirkstoffanteil 1 - 60 Gew.-%, vorzugsweise 5 - 20 Gew.-% beträgt.

4. Mittel gemäß einem der Ansprüche 1 bis 3, worin als penetrationsförderndes Mittel ein penetrationsförderndes Öl in Gewichtsteilen von 5 - 95 % zusammen mit Hilfsmitteln, wie Emulgatoren, Netzmittel und/oder Dispergatoren in Gewichtsteilen von 0,5 - 30 % und Entschäumungsmitteln in Gewichtsteilen von 3 - 60 % eingesetzt werden.

5. Mittel gemäß einem der Ansprüche 1 bis 4, enthaltend den Wirkstoff Fenbendazol.

6. Mittel gemäß einem der Ansprüche 1 bis 5 zur transdermalen Anwendung bei der Kontrolle von Helminthen bei Tieren.

7. Verfahren zur Herstellung eines Mittels gemäß einem der Ansprüche 1 bis 6, dadurch gekennzeichnet, daß man den Wirkstoff feinteilig in eine geeignete Darreichungsform bringt.

8. Derivate von Benzimidazolen, Benzthiazolen oder Pro-Benzimidazolen in feinteiliger Form zu transdermalen Anwendung bei der Kontrolle von Helminthen bei Tieren.

9. Verwendung eines Mittels einer Verbindung gemäß Anspruch 8 in feinteiliger Form bei der Herstellung einer Zubereitung zur transdermalen Behandlung von Tieren.

**Patentansprüche für folgende Vertragsstaaten : ES, GR**

1. Verfahren zur Herstellung einer anthelminthisch wirksamen Zubereitung, enthaltend als Wirkstoff mindestens ein anthelminthisch wirksames Benzimidazol, Benzthiazol oder Pro-Benzimidazol, dadurch

gekennzeichnet, daß man den Wirkstoff feinteilig in einem penetrationsfördernden Mittel feinteilig dispergiert und dann in eine geeignete Darreichungsform bringt.

**2.** Verfahren gemäß Anspruch 2, dadurch gekennzeichnet, daß die Wirkstoffteilchen in der Dispersion eine Korngröße von 0,05 - 30 µm aufweisen.

**3.** Verfahren gemäß Anspruch 1 oder 2, dadurch gekennzeichnet, daß der Wirkstoffanteil 1 - 60 Gew.-%, vorzugsweise 5 - 20 Gew.-% beträgt.

**4.** Verfahren gemäß einem der Ansprüche 1 bis 3, dadurch gekennzeichnet, daß als penetrationsförderndes Mittel ein penetrationsförderndes Öl in Gewichtsteilen von 5 - 95 % zusammen mit Hilfsmitteln, wie Emulgatoren, Netzmittel und/oder Dispergatoren in Gewichtsteilen von 0,5 - 30 % und Entschäumungsmitteln in Gewichtsteilen von 3 - 60 % eingesetzt werden.

**5.** Verfahren gemäß einem der Ansprüche 1 bis 4, dadurch gekennzeichnet, daß als Wirkstoff Fenbendazol eingesetzt wird.

**6.** Verfahren gemäß einem der Ansprüche 1 bis 5, dadurch gekennzeichnet, daß eine Zubereitung zur transdermalen Anwendung bei der Kontrolle von Helminthen bei Tieren hergestellt wird.

**7.** Verwendung von Derivaten von Benzimidazolen, Benzthiazolen oder Pro-Benzimidazolen in feinteiliger Form zur Herstellung einer Zubereitung zur transdermalen Anwendung bei der Kontrolle von Helminthen bei Tieren.

Europäisches Patentamt

# EUROPÄISCHER RECHERCHENBERICHT

Nummer der Anmeldung

EP 92 10 0818

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X<br>Y | EP-A-0 084 516 (CIBA-GEIGY AG)<br>* Seite 1, Absatz 1 *<br>* Seite 4, Absatz 2 *<br>* Seite 8, Absatz 3 *<br>* Seite 9; Beispiel 1 *<br>* Ansprüche 3,6-8 *<br>--- | 1-3,5-9<br>4 | A61K9/00 |
| X | US-A-4 018 932 (SPICER L.D. ET AL)<br>* Spalte 1, Zeile 34 - Zeile 36 *<br>* Spalte 2, Zeile 7 - Zeile 16 *<br>* Spalte 2, Zeile 45 - Spalte 3, Zeile 2 *<br>* Spalte 3 - Spalte 4; Beispiele 4,9 *<br>--- | 1,3,5-9 | |
| O,X | EP-A-0 137 627 (ICI AUSTRALIA LTD)<br>* Seite 5, Zeile 17 - Zeile 28 *<br>* Seite 7, Zeile 24 - Seite 8, Zeile 6 *<br>* Seite 11, Zeile 6 - Zeile 33 *<br>* Seite 17; Beispiel 19 *<br>--- | 1,3,6-9 | |
| X | EP-A-0 156 277 (BAYER AG)<br>* Seite 1, Zeile 1 - Zeile 3 *<br>* Seite 3, Zeile 15 - Seite 5, Zeile 15 *<br>* Seite 8; Beispiel 2 *<br>--- | 1,3,6-9 | RECHERCHIERTE SACHGEBIETE (Int. Cl.5)<br>A61K |
| Y | EP-A-0 120 286 (THE WELLCOME FOUNDATION LTD)<br>* Seite 1, Absatz 1 *<br>* Seite 1, Absatz 4 - Seite 2, Absatz 1 *<br>* Seite 2, Absatz 5 *<br>* Seite 12; Beispiel 2 *<br>--- | 4 | |
| Y | EP-A-0 054 205 (BAYER AG)<br>* Seite 1, Zeile 1 - Zeile 4 *<br>* Seite 9; Beispiel 1 *<br>--- | 4 | |
| Y | EP-A-0 045 424 (BAYER AG)<br>* Seite 1, Absatz 1 *<br>* Seite 13; Beispiele 1,3 *<br>----- | 4 | |

Der vorliegende Recherchenbericht wurde für alle Patentansprüche erstellt

| Recherchenort | Abschlußdatum der Recherche | Prüfer |
|---|---|---|
| DEN HAAG | 26 MAERZ 1992 | BOULOIS D. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument